# EUROPEAN PATENT APPLICATION

(11) **EP 2 415 433 A1**
(43) Date of publication of application: **08.02.2012**
(21) Application number: 10758626.5
(22) Date of filing: 29.03.2010
(51) Int. Cl.: A61F 13/15, A61F 13/49, A61F 13/494

(54) **DEVICE FOR PRODUCING ABSORBENT ARTICLE AND METHOD FOR PRODUCING ABSORBENT ARTICLE**

(30) Priority: 03.04.2009 JP 2009091501
(71) Applicant: Uni-Charm Corporation, Ehime 799-0111 (JP)
(72) Inventor: HAMADA, Akira, Kanonji-shi Kagawa 769-1602 (JP); OONISHI, Hidetoshi, Kanonji-shi Kagawa 769-1602 (JP); ISHIKAWA, Yasuyuki, Kanonji-shi Kagawa 769-1602 (JP)
(74) Representative: Peter, Julian
(86) International application number: PCT/JP2010/055540
(87) International publication number: WO 2010/113855

(57) **Abstract**

An apparatus for manufacturing an absorbent article, the absorbent article including an absorbent core and a sheet whereon the absorbent core is placed, including: (A) a transportation section transporting in a transport direction the sheet whereon the absorbent core is placed; and (B) a bending section bending the sheet, in an intersecting direction that intersects the transport direction, while the transportation section is transporting the sheet, (C) a first part of the sheet, positioned in a bending point where the bending section bends in the intersecting direction the sheet, is distant in the intersecting direction from the absorbent core, (D) a second part of the sheet positioned on an opposite side of the first part from the absorbent core, in the intersecting direction, collapses onto a third part positioned on the absorbent core side from the first part, in the intersecting direction, by the bending section bending the sheet at the bending point, (E) the bending section further including a regulation section regulating an approach of the first part to the absorbent core, in the intersecting direction, when the bending section is bending the sheet.

## Description

### Technical Field

The present invention relates to an apparatus and a method for manufacturing an absorbent article. Particularly, the present invention relates to an apparatus and a method for manufacturing an absorbent article, in which the absorbent article includes an absorbent core and a sheet whereon the absorbent core is placed.

### Background Art

Absorbent articles such as diapers, sanitary napkins, and the like are already well known. Also absorbent articles having an absorbent core and a sheet whereon the absorbent core is placed is well known. In a production line of the absorbent article, for example, while transporting the sheet whereon the absorbent core is placed in a transport direction, a process of bending the sheet is performed in an intersecting direction that intersects the transport direction. This process, for example, is performed to have a predetermined function imparted in a bended part of the sheet by the time of the completion phase in which the absorbent article is completed as a finished product (for example, refer to PTL 1).

### Citation List

### Patent Literature

PTL 1: JP-A-H11-506965

### SUMMARY OF INVENTION

### Technical Problem

By the way, of the sheet, in a case where a part positioned in a bending point where the sheet is bended is referred to as a first part, a part positioned on an opposite side of the first part from the absorbent core in the intersecting direction is referred to as a second part, and a part positioned nearer to the absorbent core than the first part in the intersecting direction is referred to as a third part, there is a case in which the first part is positioned distant from the absorbent core in the intersecting direction. Also, there is a case in which the sheet is bended at the bending point so that the second part collapses onto the third part. In other words, there is a case in which the sheet is bended at the bending point distant from the absorbent core so that a space is formed between the sheet and the absorbent core.

However, in the case of bending the sheet as above, since a portion of the sheet distant from the absorbent core has lower rigidity compared to a portion in which the absorbent core is positioned (a portion on which the absorbent core is placed), there is a case in which the first part is drawn toward the absorbent core (approaches the absorbent core). As a result, the above-mentioned space cannot be secured between the sheet and the absorbent core, and there is fear that the function which is to be provided to the sheet cannot be imparted appropriately.

The present invention was made in view of the foregoing issue, and it is an advantage thereof to bend the sheet appropriately while securing space between the sheet and the absorbent core, in a case where bending of the sheet at the bending point is distant from the absorbent core.

### Solution to Problem

A main aspect of the invention for solving the foregoing issue is An apparatus for manufacturing an absorbent article, the absorbent article including an absorbent core and a sheet whereon the absorbent core is placed, including: (A) a transportation section transporting in a transport direction the sheet whereon the absorbent core is placed ; and (B) a bending section bending the sheet, in an intersecting direction that intersects the transport direction, while the transportation section is transporting the sheet , (C) wherein a first part of the sheet, positioned in a bending point where the bending section bends in the intersecting direction the sheet, is distant in the intersecting direction from the absorbent core, (D) a second part of the sheet positioned on an opposite side of the first part from the absorbent core, in the intersecting direction, collapses onto a third part positioned on the absorbent core side from the first part, in the intersecting direction, by the bending section bending the sheet at the bending point, (E) the bending section further including a regulation section regulating an approach of the first part to the absorbent core, in the intersecting direction, when the bending section is bending the sheet.

Other features of the invention will become clear by the description of the present specification and the accompanying drawings.

### Advantageous Effects of Invention

According to this invention, it is possible to bend the sheet appropriately while securing space between the sheet and the absorbent core, in a case of bending the sheet at the bending point distant from the absorbent core.

### BRIEF DESCRIPTION OF DRAWINGS

FIG. 1A is a lateral view of a diaper 1.
FIG. 1B is a rear view of the diaper 1.
FIG. 1C is a view showing the diaper 1 in a spread out state.
FIG. 2 is a cross-sectional view at a center of an absorbent main body 2 in the longitudinal direction.
FIG. 3 is a cross-sectional view showing three-dimensional gathered parts 13 of the diaper 1 when worn.
FIG. 4 is a block diagram showing the main components of an apparatus for manufacturing a diaper 30.
FIG. 5 is a diagram showing a continuous body of diapers 15.
FIG. 6A is a first diagram showing a process of manufacturing the diaper 1.
FIG. 6B is a second diagram showing a process of manufacturing the diaper 1.
FIG. 6C is a third diagram showing a process of manufacturing the diaper 1.
FIG. 7 is a flowchart of a manufacturing process of the absorbent main body.
FIG. 8 is a diagram showing the process of generating a complex 22.
FIG. 9A is a cross-sectional view showing the complex 22 after a first fold-back process.
FIG. 9B is a cross-sectional view showing the complex 22 after a second fold-back process.
FIG. 9C is a cross-sectional view showing the complex 22 after a bending process.
FIG. 10A is a schematic diagram showing the flow of the bending process.
FIG. 10B is a schematic diagram showing the flow of the bending process.
FIG. 11 is a plan view showing equipments involved in the bending process of an absorbent main body manufacturing unit 31.
FIG. 12 is a lateral view of a transportation belt 42.
FIG. 13 is a view showing how the bending process is performed by a bending jig 45.
FIG. 14 is a diagram showing a comparative example for describing the effectiveness of the present embodiment.
FIG. 15 is a diagram showing a first modification example.
FIG. 16 is a diagram showing a second modification example.

### DESCRIPTION OF EMBODIMENTS

At least the following matters will be made clear by the description in the present specification and the accompanying drawings.

First, the present invention is an apparatus for manufacturing an absorbent article, the absorbent article including an absorbent core and a sheet whereon the absorbent core is placed, including:
(A) a transportation section transporting in a transport direction the sheet whereon the absorbent core is placed ; and
(B) a bending section bending the sheet, in an intersecting direction that intersects the transport direction, while the transportation section is transporting the sheet ,
(C) wherein a first part of the sheet, positioned in a bending point where the bending section bends in the intersecting direction the sheet, is distant in the intersecting direction from the absorbent core,
(D) a second part of the sheet positioned on an opposite side of the first part from the absorbent core, in the intersecting direction, collapses onto a third part positioned on the absorbent core side from the first part, in the intersecting direction, by the bending section bending the sheet at the bending point,
(E) the bending section further including:
   a regulation section regulating an approach of the first part to the absorbent core, in the intersecting direction, when the bending section is bending the sheet.

According to this apparatus for manufacturing an absorbent article, in a case of bending the sheet at the bending point distant from the absorbent core, the sheet can be bended appropriately while securing a space between the sheet and the absorbent core.

Further, in the apparatus for manufacturing an absorbent article described above, it is preferable that
the transportation section transports the sheet on a carrying face whereon the sheet is placed ,
the bending section bends at the bending point the sheet placed on the carrying face, and
the regulation section is a suction mechanism that performs suction operation to intimately attach to the carrying face the sheet, between the first part and the third part in the intersecting direction.

According to such structure, approach of the first part to the absorbent core can be regulated appropriately by using a suction force of the suction mechanism.

Further, in the apparatus for manufacturing an absorbent article described above, it is preferable that
the absorbent article has a film positioned between the absorbent core and the sheet in a thickness direction of the absorbent article,
the transportation section transports the sheet in a state where the absorbent core is placed on the sheet with the film therebetween, and
the suction mechanism performs the suction operation with the film provided between the first part and the third part, in the intersecting direction.

According to such structure, of the sheet, the area between the first part and the third part in the intersecting direction can be easily brought into intimate contact with the carrying face, and it is possible to regulate the approach of the first part to the absorbent core more efficiently.

Further, in the apparatus for manufacturing an absorbent article described above, it is preferable that
the transportation section transports the sheet on a carrying face whereon the sheet is to be placed,
the bending section bends at the bending point the sheet placed on the carrying face, and
the regulation section is a pressing body that contacts the sheet to press the sheet against the carrying face, between the first part and the third part in the intersecting direction.

According to such structure, it is possible to regulate the approach of the first part to the absorbent core efficiently by using pressing force of the pressing body.

Further, in the apparatus for manufacturing an absorbent article described above, it is preferable that
the first part and another first part are positioned on either end sides of the sheet, the second part and another second part are positioned outside the first parts, and the third part is positioned between the first parts, in the intersecting direction,
the absorbent core is provided on the third part,
two bending sections are provided, where one bending section bends the sheet at a bending point on a one end side so that the second part positioned on the one end side in the intersecting direction, of the two second parts, collapses onto the third part and
another bending section bends the sheet at the bending point on an other end side so that the second part positioned on the other end side collapses onto the third part,
two regulation sections are provided, where one regulation section regulates an approach of the first part positioned at the bending point on the one end side to the absorbent core, in the intersecting direction, when the one bending section bends the sheet at the bending point on the one end side and
another regulation section regulates an approach of the first part positioned at the bending point on the other end side to the absorbent core, in the intersecting direction, when the another bending section bends the sheet at the bending point on the other end side.

According to such structure, the regulation sections can place regulation on two of the first parts individually. As a result, adjustment according to the regulation can be made to each of the two first parts individually.

Further, it is possible to realize a method for manufacturing an absorbent article, the absorbent article including an absorbent core and a sheet whereon the absorbent core is placed, including:
(A) transporting in a transport direction the sheet whereon the absorbent core is placed; and
(B) bending the sheet, in an intersecting direction that intersects the transport direction, while transporting the sheet,
(C) a first part of the sheet, positioned in a bending point when bending the sheet in the intersecting direction, is distant in the intersecting direction from the absorbent core,
(D) the sheet is bent at the bending point so that a second part positioned on an opposite side of the first part from the absorbent core, in the intersecting direction, collapses onto a third part positioned on the absorbent core side from the first part, in the intersecting direction, when bending the sheet, and
(E) an approach of the first part to the absorbent core, in the intersecting direction, is regulated when bending the sheet.

According to the method for manufacturing an absorbent article, a sheet can be bended appropriately while securing a space between the sheet and the absorbent core in a case a sheet is bent at a bending point distant from the absorbent core.

### === Absorbent Article of the Present Invention ===

In the present embodiment, an apparatus and a method for manufacturing a diaper 1 will be explained by using the diaper 1 as an example of an absorbent article. That is, an apparatus for manufacturing a diaper 30 described later corresponds to an example of the apparatus for manufacturing an absorbent article, and the method for manufacturing the diaper 1 corresponds to an example of a method for manufacturing the absorbent article.

### « Configuration of Diaper 1 »

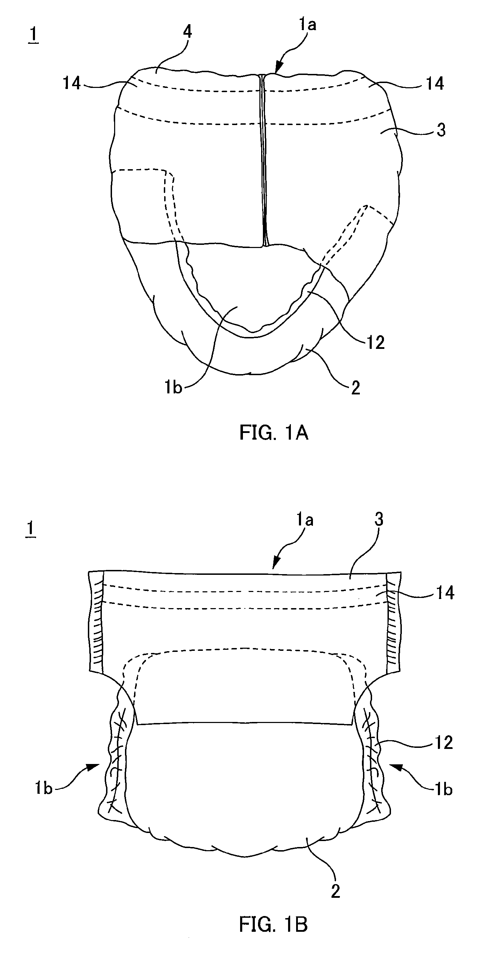

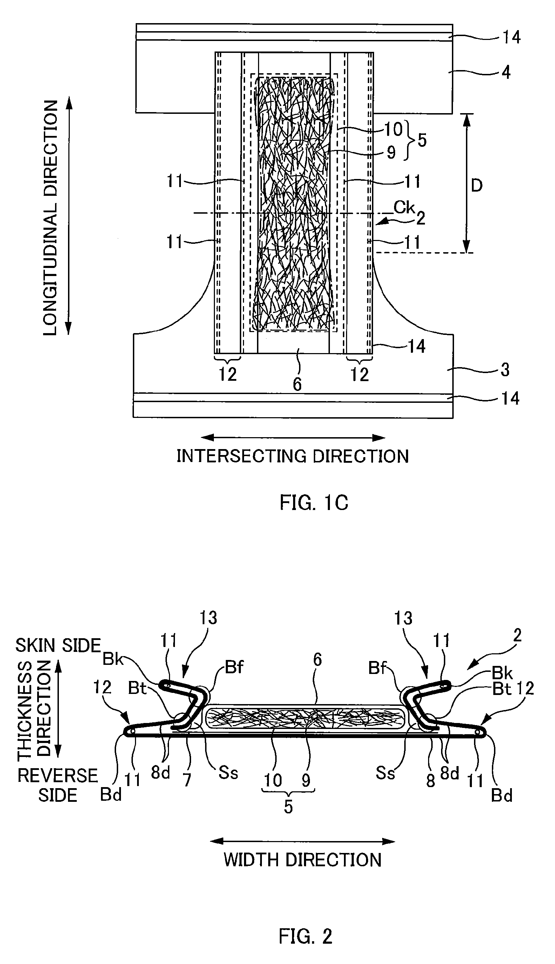

First, the configuration of the diaper 1 is explained based on FIGS. 1A to 1C and FIG. 2. FIG. 1A is a lateral view of the diaper 1, and FIG. 1B is a rear view thereof. FIG. 1C is a developed view of the diaper 1 (view from a side that contacts wearer's skin). FIG. 2 is a view showing a cross section at a center of an absorbent main body 2 in the longitudinal direction. In FIGS. 1C and 2, the longitudinal direction of the absorbent main body 2, an intersecting direction that intersects the longitudinal direction (hereafter, referred to as the width direction), and a thickness direction are each shown by an arrow.

The diaper 1 is provided with the absorbent main body 2 that absorbs body fluid such as urine by being applied to a wearer's crotch, and a pair of bands 3, 4 covering a back-side part and a stomach-side part of the wearer. In a spread out state shown in FIG. 1C, the pair of bands 3, 4 are arranged in parallel by leaving a gap D between them, and the absorbent main body 2 being bridged therebetween to make the profile substantially an H-shape seen in plan view. From such state, the diaper 1 is folded in two at a folding position Ck positioned at the center in the longitudinal direction of the absorbent main body 2. The bands 3, 4 opposing each other in the two-folded state are connected circularly by being fastened at portions that comes into contact with the wearer's sides. Thereby, the diaper 1 will be in a wearable state in which a waistline opening 1a and a pair of leg circumferential openings 1b are formed (refer to FIGS. 1A and 1B).

As the above fastening mechanism, by using a non-removable joining mechanism such as welding, a shorts-type product is formed and by using a detachable joining mechanism such as a fastening tape member (not shown), an open-type product can be formed. Further, in FIGS. 1A and 1B, the shorts-type product is exemplified. Hereafter, components of the diaper 1 will be explained.

The absorbent main body 2 is a member with a substantially rectangular plan view. As shown in FIG. 2, the center part is somewhat projected toward the side of a skin (side that contacts the wearer's skin in the thickness direction) than the vicinity thereof. The main components of the absorbent main body 2 are, as shown in FIG. 2, an absorbent body 5, a surface sheet 6 covering the absorbent body 5 from the skin side (top sheet), a back face sheet 7 covering the absorbent body 5 from an opposite side (back side) of the surface sheet 6 (back sheet), and an exterior sheet 8 (outer sheet) forming an exterior of the diaper 1 further to the reverse side of the back face sheet 7.

The absorbent body 5 consists of an absorbent core 9 formed by molding liquid absorbent fiber such as pulp fiber into a substantially guitar-shape plan view, and a tracing paper 10 such as tissue that wraps the absorbent core 9. The absorbent core 9 can include superabsorbent polymer (SAP). The surface sheet 6 is a permeable nonwoven fabric. The back face sheet 7 is an impermeable film sheet as an example of a film sheet of the invention. Each of the surface sheet 6 and the back face sheet 7 has a planar size that is larger than the absorbent body 5, and the back face sheet 7 is slightly wider than the surface sheet 6. The absorbent body 5 is disposed at a center part of the absorbent main body 2 by being sandwiched between the surface sheet 6 and the back face sheet 7. The above two sheets 6 and 7 are bonded together at parts protruding outside from the four sides of the absorbent body 5 to form a frame-shape.

The exterior sheet 8 is an example of a sheet of the invention, and is a nonwoven fabric sheet. The exterior sheet 8 has a planar size larger than the surface sheet 6 and the back face sheet 7, and on a skin-side face thereof the absorbent body 5 is placed by being sandwiched between the above two sheets 6 and 7. That is, the exterior sheet 8 is a sheet whereon the absorbent core 9 wrapped by the tracing paper 10 is placed with the back face sheet 7 placed therebetween. In other words, in the thickness direction of the absorbent main body 2, the back face sheet 7 lies between the absorbent core 9 wrapped by the tracing paper 10 and the exterior sheet 8. As shown in FIG. 2, parts positioned on the end sides in the width direction of the exterior sheet 8 are folded into a substantially Z-shape, and such folded parts are symmetrically provided with respect to the center in the width direction of the exterior sheet 8.

Specifically describing, of the exterior sheet 8, a part that is elongated toward the outside in the width direction is folded back inwards, and parts overlapping in the vicinity of a fold-back point Bd (hereafter, overlapping part 8d) are joined together. Also, in the vicinity of the fold-back point Bd, a stretchable member 11 such as an elastic cord is fixed along the longitudinal direction of the absorbent main body 2 in an expanded state. In this way, at parts sandwiching the absorbent body 5 in the width direction of the exterior sheet 8, gathered parts at the leg circumference 12 imparted with stretch properties are formed at the leg circumferential openings 1b of the diaper 1.

Also, as shown in FIG. 2, in the exterior sheet 8, the part folded back at the fold-back point Bd is folded back again at a second fold-back point Bk positioned at a midway position thereof, and further crooked to somewhat stand up at a stand-up point Bt positioned between the fold-back point Bd and the second fold-back point Bk. This stand up portion is, as shown in FIG. 2, reclining onto the absorbent body 5 (strictly speaking, the surface sheet 6 covering the absorbent body 5) and folded back again toward outside in the width direction at a third fold-back point Bf. In a vicinity of a leading end of the folded part (a part at which the second fold-back point Bk is positioned), the stretchable member 11 is fixed along the longitudinal direction of the absorbent main body 2, while being wrapped by the exterior sheet 8, in an expanded state. Thereby, in the exterior sheet 8, three-dimensional gathered parts 13 are formed at parts positioned on both sides of the absorbent body 5.

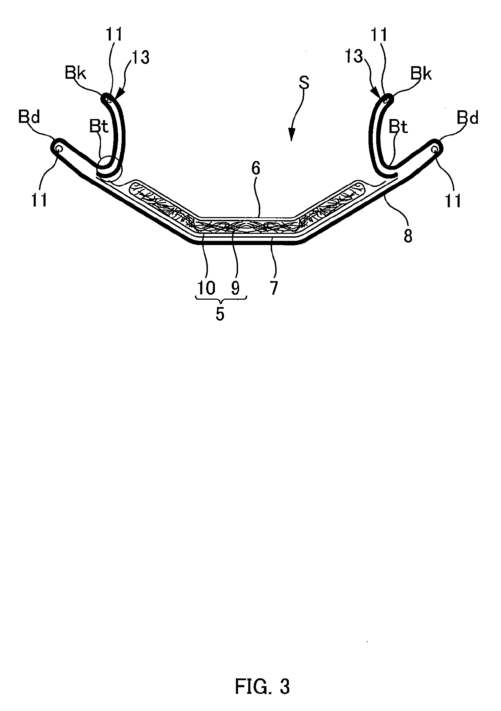

As shown in FIG. 3, the three-dimensional gathered parts 13 stand up in a case where the diaper 1 is in a worn state, and reflexes with the stand-up point Bt as a fulcrum to contact a vicinity of the wearer's groin, and thereby an accommodating space S for accommodating excrements is formed between the three-dimensional gathered parts 13. FIG. 3 is a cross-sectional view showing the three-dimensional gathered parts 13 in a state when the diaper 1 is worn. As shown in the figure, the width of the accommodating space S (length in the width direction) is somewhat longer than the width of the absorbent core 9. That is, a space between the stand-up points Bt in the width direction is wider than the width of the absorbent core 9. Positional relationship between the exterior sheet 8 and the absorbent core 9 will be described later.

The pair of bands 3, 4 are flexible sheets such as nonwoven fabric, and they are cut into a substantially rectangular shape in a planar view and intersect (intersect at substantially right angles) the longitudinal direction of the absorbent main body 2. And in the center parts of the bands 3 and 4 in the longitudinal direction, the end parts in the longitudinal direction of the absorbent main body 2 bridged between the bands 3 and 4 are bonded and fixed. Further, in a case where each band 3 and 4 consists of two-ply nonwoven fabrics, the end parts in the longitudinal direction of the absorbent main body 2 can be fixed by being sandwiched between the nonwoven fabrics. Also, it is possible that an elastic member 14 such as an elastic cord or an elastic band (refer to FIG. 1C) is fixed to each band 3 and 4 along the longitudinal direction of the bands 3 and 4 in an expanded state, to provide stretch properties to each band 3 and 4. Further, it is possible to perform die-cutting to the corner parts of the bands 3 and 4 so as to form a substantially fan-shape, so that the fitting of the parts of the bands 3 and 4 forming the leg circumferential openings 1b to the wearer's thigh is improved. In the present embodiment, of the bands 3 and 4, the die-cutting is performed only to the band 3 that covers the back-side part of the wearer (back-side band 3). However there is no limitation to this. And the die-cutting can be performed to the band 4 that covers the stomach-side part (stomach-side band 4).

### « Positional Relationship between Exterior Sheet 8 and Absorbent Core »

Next, the positional relationship between the exterior sheet 8 and the absorbent core, mainly, the positional relationship in the width direction of the absorbent main body 2 is explained.

The exterior sheet 8 is folded back at each of the fold-back points Bd, Bk, and Bf mentioned above with a part of the exterior sheet 8 made to stand up at the stand-up point Bt to recline onto both end parts in the width direction of the absorbent body 5 (refer to FIG. 2). And by fixing the stretchable member 11 in the vicinity of each of the fold-back point Bd and the second fold-back point Bk in an expanded state, the gathered part in leg circumference 12 and the three-dimensional gathered part 13 are formed at predetermined portions of the exterior sheet 8.

By the way, the stand-up point Bt is positioned outside the end of the absorbent body 5 in the width direction (refer to FIG. 2). That is, the stand-up point Bt is positioned in a position distant from the absorbent core 9 in the width direction. As a result, in the width direction, a space Ss is formed between the exterior sheet 8 standing up from the stand-up point Bt and the absorbent core 9 (refer to FIG. 2).

The main reasons for configuring the stand-up point Bt in the above position are, to make the height of the three-dimensional gathered part 13 that reflexes with the stand-up point Bt as a fulcrum when wearing the diaper 1 as high as possible, and to make the capacity of the accommodating space S for accommodating excrements formed between the solid gathered parts 13 as large as possible. That is, if the standing-up point Bt is far from the absorbent core 9 in the width direction (if the space Ss is formed between the exterior sheet 8 and the absorbent core 9), the height of the three-dimensional gathered part 13 in the reflexed state (state shown in FIG. 3) becomes higher and the capacity of the accommodating space S becomes larger. From the above reason, in the embodiment, in the exterior sheet 8, an inner end of a part at which the gathered part in leg circumference 12 is formed (overlapping part 8d overlapping in the vicinity of the fold-back point Bd) in the width direction is the stand-up point Bt.

In the present embodiment, an end of the back face sheet 7 in the width direction is about to reach the stand-up point Bt (refer to FIG. 2). That is, of the skin-side face of the exterior sheet 8, a region positioned between the stand-up points Bt in the width direction is covered with the back face sheet 7.

### « Method for Manufacturing Diaper 1 »

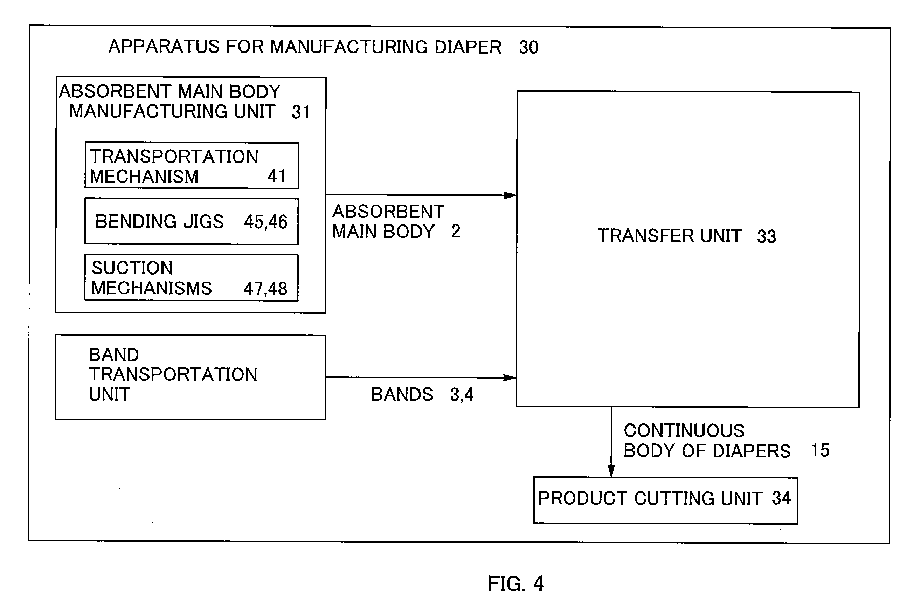

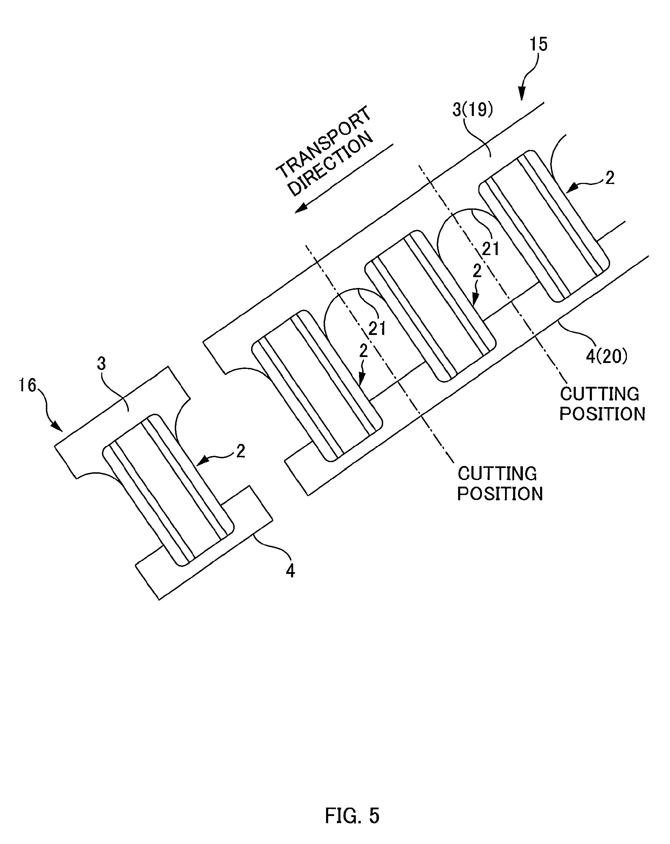

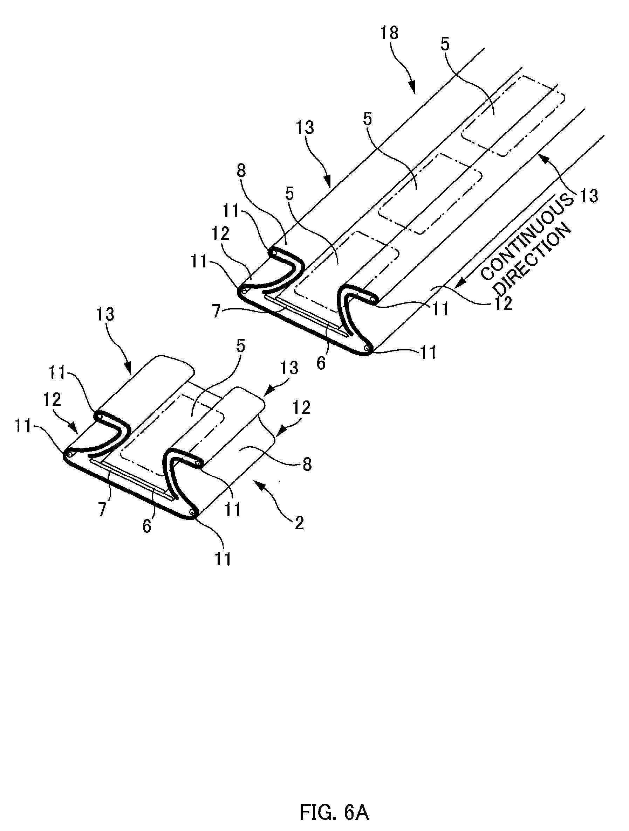

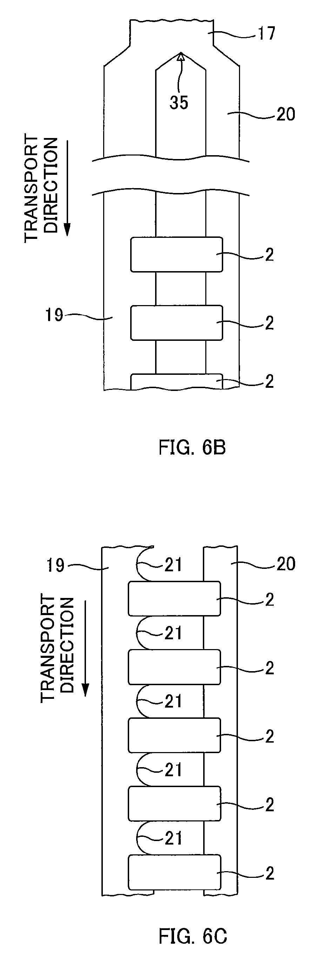

Next, by referring to FIGS. 4 to 6C, a method for manufacturing the diaper 1 is summarized. FIG. 4 is a block diagram showing the main components of an apparatus for manufacturing a diaper 30. FIG. 5 is a diagram showing a continuous body of diapers 15. FIGS. 6A to 6C are diagrams showing manufacturing processes of the diaper 1. The absorbent main body 2 is described in a simplified manner in FIGS. 6B and 6C for the sake of simplification.

The method for manufacturing the diaper 1 according to the present embodiment is a method for continuously manufacturing the diaper 1 as a product and it is performed by the apparatus for manufacturing a diaper 30. As shown in FIG. 4, the apparatus for manufacturing a diaper 30 includes an absorbent main body manufacturing unit 31, a band supplying unit 32, a transfer unit 33, and a product cutting unit 34 etc.

The absorbent main body manufacturing unit 31 is a unit that performs process of manufacturing the absorbent main body 2 by combining each component that constitutes the absorbent main body 2 (hereafter, referred to as the absorbent main body manufacturing process). In the absorbent main body manufacturing process, each of the surface sheet 6, the back face sheet 7, and the exterior sheet 8 is reeled out from a rolled up state and the sheets are joined together while sandwiching the absorbent body 5 (specifically, the absorbent core 9 wrapped by the tracing paper 10) between the surface sheet 6 and the back face sheet 7. Further, each of the sheets 6, 7, and 8 is reeled out in a continuous state, and the absorbent body 5 is disposed at a predetermined interval in the continuous direction. As a result, the absorbent main bodies 2 are formed so as to continue along the longitudinal direction and thus forms a continuous body 18 (refer to FIG. 6A). The continuous body 18 is transported to the following transfer process in the continuous state, and cut into units of products and thereby divided into individual absorbent main bodies 2 at the start of the transfer process.

Also, during the absorbent main body manufacturing process, the exterior sheet 8 is folded back at each of the fold-back points Bd, Bk, and Bf and a portion thereof is made to stand up at the stand-up point Bt, while the stretchable member 11 is fixed to the predetermined portion of the exterior sheet 8 along a continuous direction of the exterior sheet 8 in a expanded state. Thus, each absorbent main body 2 constituting the continuous body 18 is transported to the transfer process with the gathered part of the leg circumference 12 and the three-dimensional gathered part 13 in a formed state. Details of the absorbent main body manufacturing unit 31 and the absorbent main body manufacturing process will be described later.

The band supplying unit 32 is a unit for supplying the bands 3 and 4 to the transfer process. As shown in FIG. 6B, each band 3 and 4 is supplied as continuous bands 19 and 20 and moves along the continuing direction. In the embodiment, as shown in the figure, a continuous band before separation 17 that is wide in some degree is split by a splitter 35 in the width direction, and thus separated into a pair of continuous bands 19 and 20. After separation, the continuous bands 19 and 20 move in a substantially parallel state with intervals corresponding to the gap D between the bands 3 and 4 of the completed product.

The transfer unit 33 is a unit for transferring, to the pair of bands 3 and 4, the absorbent main bodies 2 as individual bodies that are obtained by cutting, into units of products, the continuous body 18 of the absorbent main bodies 2 transported from the absorbent main body manufacturing process, where the pair of bands 3 and 4 are supplied in the state of continuous bands 19 and 20 by the band supplying unit 32. As shown in FIG. 6B, the transfer unit 33 places the absorbent main bodies 2, so that the absorbent main bodies 2 are bridged between the bands 3 and 4 supplied in the state of the continuous bands 19 and 20, at predetermined intervals along the continuous direction of the continuous bands 19 and 20. At this time, each of the absorbent main bodies 2 are joined to each of the bands 3 and 4 at end parts thereof in the longitudinal direction. And as a result of joining the plurality of absorbent main bodies 2 to the bands 3 and 4 sequentially, the pair of bands 3 and 4 and the absorbent main bodies 2 bridged therebetween form a shape of a ladder.

Thereafter, each of the bands 3 and 4 is continuously transported in the transport direction, and during such transportation, a die-cutting process is performed to the continuous band 19 of the back-side band 3. The die-cutting process is a process of cutting a portion positioned between the adjacent absorbent main bodies 2 of the continuous band 19 of the back-side band 3, and is performed by a die-cutting unit not shown. By performing the die-cutting process, the continuous band 19 of the back-side band 3 is cut in a substantially semicircular shape at each predetermined interval along the continuous direction thereof, whereby a substantially arc-shaped arch 21 is formed at each cut out portions (refer to FIG. 6C).

By the above mentioned processes, a continuous body of diapers 15 is formed in which the diapers 1 in spread out states are connected in series in the transport direction (refer to FIG. 5). The continuous body of diapers 15 is transported to the following product cutting process in the state of being connected in series. In the product cutting process, the product cutting unit 34 cuts the continuous body of diapers 15 into units of products by a cutter not shown. And by performing a finishing process to a piece of continuous body of diapers 16 (refer to FIG. 5) that has been cut, (for example, in a case of the shorts-type product, the piece of continuous body of diapers 16 is folded in two at a folding position Ck, and the bands 3 and 4 are circularly connected by welding etc. and thus bonded) the diaper 1 as a product is completed.

### === Absorbent Main Body Manufacturing Process by Absorbent Main Body Manufacturing Unit 31 ===

In this chapter, the absorbent main body manufacturing unit 31 and the absorbent main body manufacturing process described before are explained in more detail.

### « Absorbent Main Body Manufacturing Process »

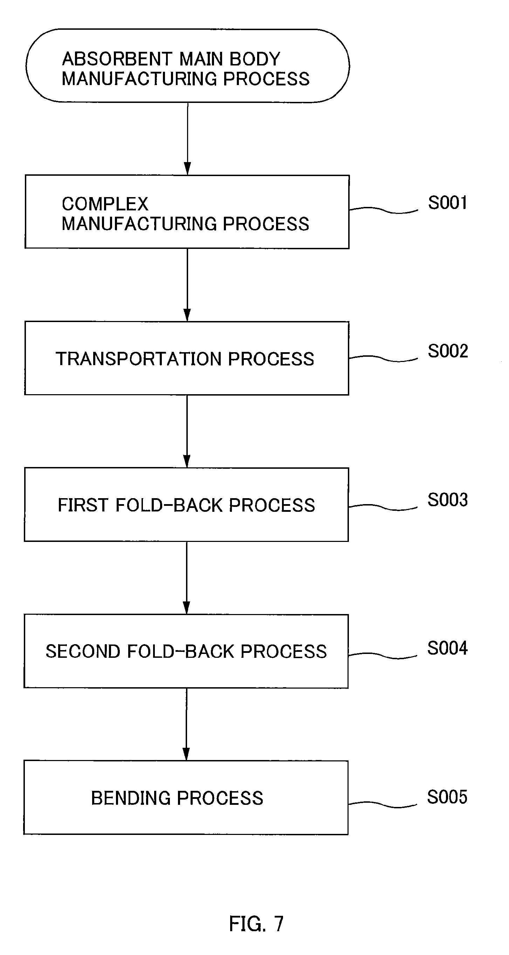

First, a broad outline of the absorbent main body manufacturing process is explained referring to FIG. 7. FIG. 7 is a flowchart of the absorbent main body manufacturing process. As shown in FIG. 7, the absorbent main body manufacturing process includes a complex manufacturing process (S001), a transportation process (S002), a first fold-back process (S003), a second fold-back process (S004), and a bending process (S005). These processes are performed in the above order.

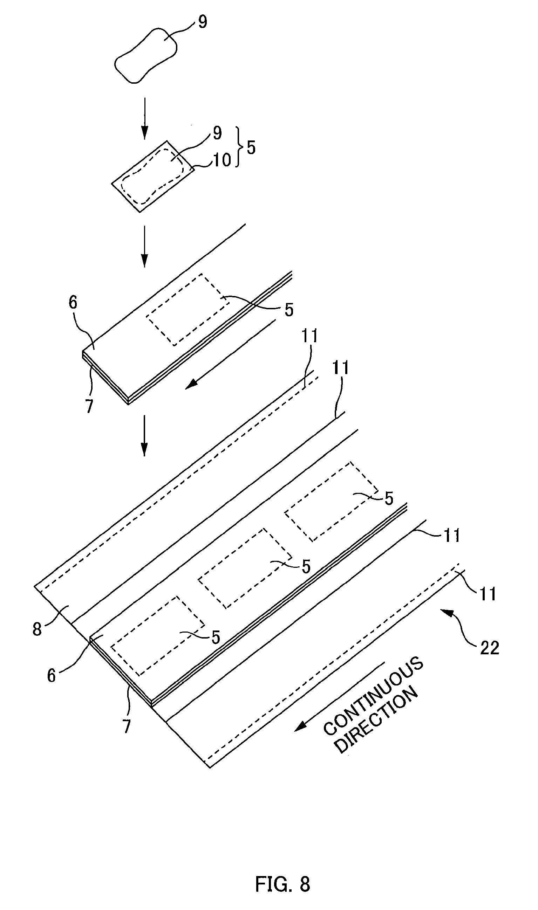

The complex manufacturing process is a process of manufacturing a complex 22 by bonding each element composing the absorbent main body 2. Specifically describing, in this process, the absorbent core 9 molded into a substantially guitar-shape is wrapped by the tracing paper 10 and thereby the absorbent 5 is created as shown in FIG. 8. After this the absorbent 5 is sandwiched between the surface sheet 6 and the back face sheet 7 and both sheets 6 and 7 are bonded, and the bonded sheets 6 and 7 are attached to the skin-side face of the exterior sheet 8 to create the complex 22 shown in FIG. 8 in the end. FIG. 8 is a diagram showing process of generating the complex 22.

Further, the surface sheet 6, the back face sheet 7, and the exterior sheet 8 are respectively charged into this process in a continuous state. And the complex 22 is generated continuously in a same direction as the continuous direction of each of the sheets 6, 7, and 8. In the exterior sheet 8, each of the stretchable member 11 forming the gathered part in leg circumference 12, and the stretchable member 11 forming the three-dimensional gathered part 13 is bonded to a predetermined bonding position, and the exterior sheet 8 is charged into the complex manufacturing process in such state. However, the stretchable member 11 can be bonded in a subsequent process, for example, just before the bending process.

The transport process is a process for transporting the complex 22 created in the previous process in the transport direction lying along the continuous direction of the complex 22. In other words, the transport process is a process in which the absorbent core 9 wrapped by the tracing paper 10 is placed on the exterior sheet 8 with the back face sheet 7 therebetween, and the exterior sheet 8 is transported in such state. This process is performed by a transportation mechanism 41 described later.

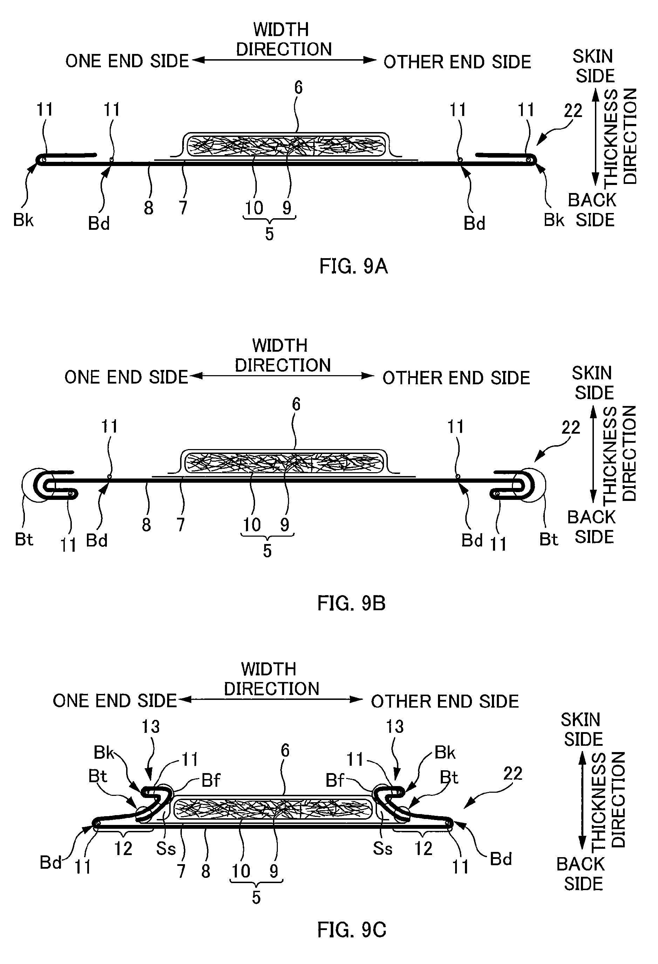

The first fold-back process is, during the transport process (that is, while the transportation mechanism 41 is transporting the exterior sheet 8), a process of folding back the exterior sheet 8 in the complex 22 at the second fold-back point Bk. By this process, the exterior sheet 8 is formed into a state shown in FIG. 9A. FIG. 9A is a cross-sectional view showing the complex 22 after the first fold-back process. As shown in FIG. 9A, in the first fold-back process, the exterior sheet 8 is folded so that the exterior sheet 8 (specifically, a part positioned in the vicinity of the second fold-back point Bk) wraps the stretchable member 11 for forming the three-dimensional gathered part 13. And after the first fold-back process, the complex 22 is transported continuously.

The second fold-back process is, during the transport process, a process of folding back the exterior sheet 8 in a state shown in FIG. 9A at the third fold-back point Bf so that both end parts in the width direction thereof turns backwards. By the process, the exterior sheet 8 shifts from the state shown in FIG. 9A to the state shown in FIG. 9B. The FIG. 9B is a cross-sectional view showing the complex 22 after the second fold-back process. The complex 22 is transported continuously while maintaining such state.

The bending process is, during the transport process, a process of folding back the exterior sheet 8 in a state shown in FIG. 9B at the fold-back point Bd, and further bending the exterior sheet 8 at the stand-up point Bt as a bending point. By this process, the exterior sheet 8 shifts from the state shown in FIG. 9B to the state shown in FIG. 9C. The FIG. 9C is a cross-sectional view showing the complex 22 after the bending processes. And in a finishing phase of the bending process, the gathered part in leg circumference 12 and the three-dimensional gathered part 13 are formed in the predetermined portions of the exterior sheet 8.

By going through the above processes, the complex 22 becomes a continuous body 18 of the absorbent main bodies 2 and thereafter, moves along the continuous direction thereof and is transported to the subsequent transfer process. Further, in each of the processes from the first fold-back process to the bending process, the folding back or bending of the exterior sheet 8 is symmetrically performed with respect to the center of the exterior sheet 8 in the width direction.

### « Regarding Bending Process »

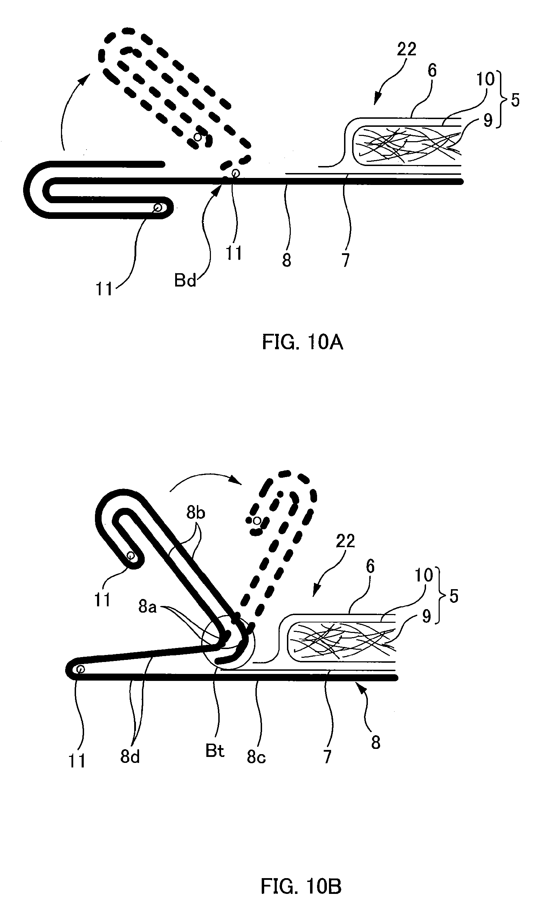

Next, the bending process mentioned above will be described in detail referring to FIGS. 10A and 10B. FIGS. 10A and 10B are schematic diagrams showing the flow of the bending process.

As described above, the bending process is performed while the transportation mechanism 41 transports the exterior sheet 8. In this process, first, as shown in FIG. 10A, the exterior sheet 8 is folded so that a part positioned outside the fold-back point Bd in the width direction collapses onto a part positioned inside the fold-back point Bd in the width direction with a part positioned at the fold-back point Bd of the exterior sheet 8 set as fulcrum. In this way, the overlapping part 8d is gradually formed from the fold-back point Bd and inward in the width direction. As the folding of the exterior sheet 8 proceeds, of the exterior sheet 8, the fulcrum switches to a part positioned in the stand-up point Bt. After that, as shown in FIG. 10B, the exterior sheet 8 is bent so as to rotate about the part positioned in the stand-up point Bt as the fulcrum, and a part positioned on the free-end side in the exterior sheet 8 reclines until it collapses onto the absorbent body 5.

As described above, in the bending process, the fulcrum switches from the part positioned in the fold-back point Bd to the part positioned in the stand-up point Bt. However, to realize the switching of the fulcrum, an adhesive such as a hot-melt adhesive is applied in advance to a part between the fold-back point Bd and the stand-up point Bt before performing the bending process. That is, by folding the exterior sheet 8 by using the part positioned in the fold-back point Bd as the fulcrum, the overlapping part 8d of the exterior sheet 8 gradually widens from the fold-back point Bd and inward in the width direction, and when reaching the stand-up point Bt, the overlapping parts 8d will be joined together up to the stand-up point Bt. With both of the overlapping parts 8d joined together up to the stand-up point Bt, the fulcrum switches and hereafter, the part positioned on the free-end side in the exterior sheet 8 rotates about that fulcrum.

However, at the point in which the fulcrum is switched to the part positioned in the stand-up point Bt, the part positioned on the free-end side of the exterior sheet 8 is positioned on the opposite side of the stand-up point Bt from the absorbent core 9 in the width direction of the exterior sheet 8 (refer to FIG. 10B).

Here, of the exterior sheet 8, a part positioned at the stand-up point Bt in the width direction is referred to as the first part 8a, a part positioned on opposite side of the first part 8a from the absorbent core 9 in the width direction is referred to as the second part 8b, and a part positioned nearer the absorbent core 9 than the first part 8a in the width direction is referred to as the third part 8c. During the bending process, at a phase of switching the fulcrum to the part positioned at the stand-up point Bt (that is, the first part 8a), the part positioned on the free-end side of the exterior sheet 8 corresponds to the second part 8b, and the part positioned inside of the stand-up point Bt in the width direction corresponds to the third part 8c. In that sense, the bending process can be referred to as a process of bending the exterior sheet 8 at the bending point (the stand-up point Bt) so that the second part 8b collapses onto the third part 8c.

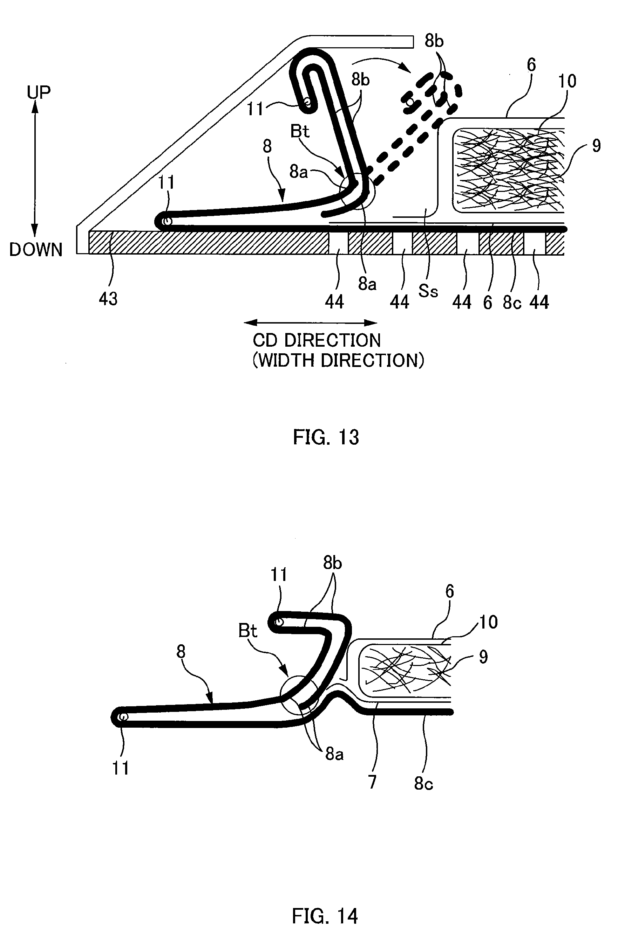

Also, during the bending process, the absorbent body 5 is disposed on a center of the third part 8c in the width direction. Therefore, the first part 8a is separated from the absorbent core 9 in the width direction. That is, in the bending process of the embodiment, the exterior sheet 8 is bended at a bending point far from the absorbent core 9 in the width direction. Thereby, in the complex 22 after the bending process, a space Ss is formed between the exterior sheet 8 and the absorbent core 9 in the width direction (refer to FIG. 13 for example).

Further, as described above, in the bending process, the exterior sheet 8 is symmetrically bended with respect to the center thereof in the width direction. That is, in the exterior sheet 8 of the complex 22 that has gone through the first fold-back process and the second fold-back process and heading for the bending process, the first part 8a is positioned on both end sides of the exterior sheet 8 in the width direction, the second part 8b is positioned outside the first part 8a in the width direction, and the third part 8c is positioned between the two first parts 8a. In such state, the exterior sheet 8 is bended symmetrically at each stand-up points Bt as the bending point. Hereafter, the stand-up point Bt positioned on one end side in the width direction is referred to as the one-end side stand-up point Bt (corresponds to an one-end side bending point), and the stand-up point Bt positioned on the other end side is referred to as the other-end side stand-up point Bt (corresponds to the other-end side bending point).

### « Regarding Absorbent Main Body Manufacturing Unit 31»

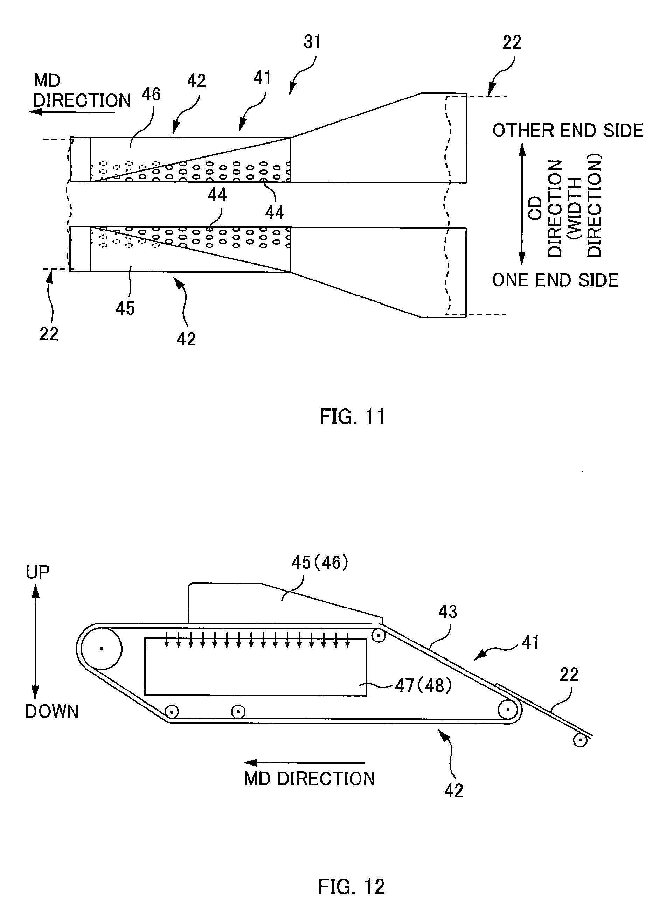

Next, of the absorbent main body manufacturing unit 31, equipments involved in the above mentioned bending process are mainly explained with reference to FIG. 11. FIG. 11 is a plan view showing the equipments involved in the bending process of the absorbent main body manufacturing unit 31. Further, in following explanation, a direction in which materials flow (for example, the transport direction of the complex 22) is referred to as the MD direction, and a direction intersecting the MD direction in a horizontal plane (orthogonal) is referred to as the CD direction.

As shown in FIG. 11, the absorbent main body manufacturing unit 31 has a transportation mechanism 41 as an example of a transportation section, bending jigs 45, 46 as examples of a bending section, and suction mechanisms 47, 48. Hereafter, these equipments are described in detail.

### < Transportation Mechanism 41 >

The transportation mechanism 41 performs the aforementioned transportation process and transports the complex 22 in the MD direction along the direction in which the complex 22 continues. As shown in FIG. 11, the transportation mechanism 41 includes a pair of transportation belts 42 arranged along the CD direction. The transportation belt 42 is a circulating belt as shown in FIG. 12, and rotates to transport the materials placed on a carrying face 43 which is a surface thereof in the MD direction. FIG. 12 is a lateral view of the transportation belt 42.

And the pair of transportation belts 42 cooperate to receive the complex 22 transported from the second fold-back process, and transports the complex 22 while opposing and contacting the exterior sheet 8, that is, a lower face of the complex 22 to the carrying face 43. That is, the carrying face 43 of the transportation belt 42 is the carrying face 43 for placing the exterior sheet 8 of the complex 22. Further, the complex 22 is transported in the MD direction in a state where the width direction thereof faces the CD direction. In other words, the width direction of the exterior sheet 8 and the other components of the complex 22 intersects (is orthogonal to) the transport direction.

Also in the present embodiment, the pair of transportation belts 42 are arranged substantially parallel with an interval therebetween. The one end part of the exterior sheet 8 in the width direction is placed on the carrying face 43 of the transportation belt 42 on the one end side of the CD direction, and the other end part of the exterior sheet 8 in the width direction is placed on the carrying face 43 of the transportation belt 42 on other end side in the CD direction.

Explaining in detail, the complex 22 including the exterior sheet 8 is received by the pair of transportation belts 42 in a state shown in FIG. 9B. And of the exterior sheet 8, the one end part of the third part 8c in the width direction and a part positioned further to the one end side are placed on the carrying face 43 of the transportation belt 42 on the one end side of the CD direction. On the other hand, of the exterior sheet 8, the other end part of the third part 8c in the width direction and a part positioned further to the other end side are placed on the carrying face 43 of the transportation belt 42 on the other end side of the CD direction.

Since there is an interval between the transportation belts 42, of the exterior sheet 8, a center part of the third part 8c in the width direction is not placed on the carrying face 43 (that is, not being supported) when transporting the complex 22. However, there is no limitation to this and it is possible that the transportation belts 42 are arranged without including any interval between them, and the center part of the third part 8c in the width direction is placed on the carrying face 43 when transporting the complex 22.

Also, the transportation mechanism 41 transports the complex 22, in other words, transports the exterior sheet 8 whereon the absorbent core 9 is placed in the MD direction (that is, the transport process is the process of transporting the exterior sheet 8 whereon the absorbent core 9 is placed in the MD direction). Also, at a phase of the transportation mechanism 41 transporting the exterior sheet 8, the exterior sheet 8 is in a state at which most part of its skin-side face is covered with the back face sheet 7. That is, the transportation mechanism 41 transports the exterior sheet 8 in a state where the absorbent core 9 is placed on the exterior sheet 8 with the back face sheet 7 therebetween. Further, the back face sheet 7 extendeds from the one-end side stand-up point Bt to the other-end side stand-up point Bt in the width direction (refer to FIG. 9C).

### < Bending Jigs 45, 46 >

The bending jigs 45 and 46 perform the bending process mentioned above and two jigs are provided in the present embodiment. Each of two jigs 45 and 46 bends the exterior sheet 8 in the CD direction intersecting the MD direction being the transport direction (in other words, the width direction of the exterior sheet 8) while the transportation mechanism 41 transports the complex 22 including the exterior sheet 8.

Specifically describing, of the two bending jigs 45 and 46, one of the bending jig 45 is engaged to the transportation belt 42 on the one end side in the CD direction (refer to FIG. 11). And the one of the bending jig 45 bends the exterior sheet 8 placed on the carrying face 43 of the transportation belt 42 at the one-end side stand-up point Bt (that is, the second part 8b positioned on the one end side in the width direction is bent so as to collapse onto the third part 8c). The other bending jig 46 is engaged to the transportation belt 42 on the other end side in the CD direction (refer to FIG. 11). And the other bending jig 46 bends the exterior sheet 8 placed on the carrying face 43 of the transportation belt 42 at the other-end side stand-up point Bt (that is, the second part 8b positioned on the other end side in the width direction is bent so as to collapse onto the third part 8c).

Since the bending jigs 45 and 46 have a substantially same structure that is symmetrical, hereafter, explanation will be given only on the bending jig 45. The bending jig 45 is a plate-like member as shown in FIG. 13. And when the complex 22 in a state shown in FIG. 9B passes the position including the bending jig 45 in the MD direction, the bending jig 45 gradually draws in the one end part of the exterior sheet 8 in the width direction. FIG. 13 is a view showing how the bending process is performed by the bending jig 45.

Specifically describing, at the point the complex 22 reaches the position provided with the bending jig 45 in the MD direction, the bending jig 45 contacts a part positioned in the vicinity of the fold-back point Bd of the exterior sheet 8. And as the complex 22 is transported to the downstream side in the MD direction, the one end part of the exterior sheet 8 in the width direction is pulled to be rolled inside, and the exterior sheet 8 is folded back with the part positioned at the fold-back point Bd as the fulcrum. Then, as the folding back of the exterior sheet 8 further proceeds, as mentioned above, the fulcrum is switched to the part positioned at the stand-up point Bt (that is, the first part 8a), and the second part 8b reclines so as to be placed onto the absorbent core 9 (that is, collapses onto the third part 8c). As a result, the complex 22 including the exterior sheet 8 will be in a state shown in FIG. 9C at the point of passing the position provided with the bending jig 45 in the MD direction, and the exterior sheet 8 is bent at the stand-up point Bt and will be in a state of somewhat standing up.

### < Suction Mechanisms 47 and 48 >

The suction mechanisms 47 and 48 perform suction operation to intimately attach a predetermined part of the exterior sheet 8 to the carrying face 43 of the transportation belt 42, during the bending process performed by the bending jigs 45 and 46. In the present embodiment, two of the suction mechanisms 47 and 48 are provided and one suction mechanism 47 is disposed inside the transportation belt 42 on the one end side in the CD direction, and other suction mechanism 48 is disposed inside the transportation belt 42 on the other end side in the CD direction. Since two suction mechanisms 47 and 48 have a similar constitution and function, hereafter the function or the like will be explained by using the one suction mechanism 47 as an example.

The suction operation performed by the suction mechanism 47 is an operation of sucking air through air holes 44 formed on the carrying face 43. Here, the air holes 44 are formed in an area, of the carrying face 43, in which the bending jig 45 is provided in the MD direction (that is, an area in which the bending process is performed). Also the air holes 44 are formed on an inner half side in the CD direction of the above mentioned area. As a result, as shown in FIG. 13, of the back side face of the exterior sheet 8 (the face contacting the carrying face 43), a part that covers an area extending from the first part 8a to an end part in the width direction of the third part 8c opposes the area in which the air holes 44 are formed to the carrying face 43, while the other parts do not oppose the area in which the air holes 44 are formed (refer to FIG. 13).

Therefore, the exterior sheet 8 is intimately attached to the carrying face 43 on which the air holes 44 are formed between the first part 8a and the third part 8c in the width direction by the suction mechanism 47 sucking air through the air holes 44. In other words, the suction operation performed by the suction mechanisms 47 and 48 is an operation for intimately attaching the exterior sheet 8 to the carrying face 43 between the first part 8a and the third part 8c in the width direction, while performing the bending process.

Further, as mentioned above, at a phase in which the exterior sheet 8 is placed on the carrying face 43, the back face sheet 7 is provided between the first part 8a and the third part 8c in the width direction. That is, the suction mechanism 47 performs the suction operation in a state in which the back face sheet 7 is provided on a part, of the exterior sheet 8, opposing the area in which the air holes 44 are formed on the carrying face 43. Since the back face sheet 7 is a film of poor air permeability, by providing the back face sheet 7 between the first part 8a and the third part 8c, the effect of intimately attaching the back side face of the exterior sheet 8 to the carrying face 43 between the first part 8a and the third part 8c in the width direction can be further enhanced.

As described above, as a result of intimately attaching the first part 8a and the third part 8c to the carrying face 43 by the suction mechanism 47 performing the suction operation, misalignment of the first part 8a and the third part 8c that can occur during the bending process can be suppressed. In this way, it is possible to regulate the first part 8a from approaching the absorbent core 9 in the width direction during the bending process, and when bending the exterior sheet 8 at the stand-up point Bt, the space Ss is appropriately secured between the exterior sheet 8 and the absorbent core 9.

That is, the suction mechanism 47 corresponds to a regulation section that regulates the approach of the first part 8a to the absorbent core 9 in the width direction by performing the above suction operation, when the bending jig 45 bends the exterior sheet 8. In other words, in the bending process of the present embodiment, approach of the first part 8a to the absorbent core 9 in the width direction is regulated when bending the exterior sheet 8.

Further, in the present embodiment, as described above, two suction mechanisms 47 and 48 are provided. In the case where the one bending jig 45 bends the exterior sheet 8 in the one-end side stand-up point Bt, the one suction mechanism 47 regulates the approach of the first part 8a positioned in the one-end side stand-up point Bt toward the absorbent core 9 in the width direction. In the case where the other bending jig 46 bends the exterior sheet 8 at the other-end side stand-up point Bt, the other suction mechanism 48 regulates the approach of the first part 8a positioned in the other-end side stand-up point Bt toward the absorbent core 9 in the width direction. In this way, by respectively providing the suction mechanisms 47 and 48 as the regulation section to each of the bending jigs 45 and 46, the suction mechanisms 47 and 48 can regualte the two first parts 8a individually. As a result, adjustment according to the regulation (for example, adjustment of suction pressure) can be made to each of the two first parts 8a individually.

### == Effectiveness of Present Embodiment ==

According to the method for manufacturing the diaper 1 and the apparatus for manufacturing the diaper 30 of the present embodiment, in the case of bending the exterior sheet 8 at the bending point distant from the absorbent core 9, the exterior sheet 8 can be bended appropriately while securing the space Ss between the exterior sheet 8 and the absorbent core 9. Hereafter, the effectiveness of the embodiment is described in detail.

As already explained in the RELATED ART, there is a case in which the exterior sheet 8 is bended at the bending point distant from the absorbent core 9 in the width direction so that space Ss is formed between the exterior sheet 8 and the absorbent core 9. For example, in the case of the present embodiment, the space Ss is necessary to make the height of the three-dimensional gathered part 13 high in the case where the three-dimensional gathered part 13 reflexes when wearing the diaper 1, and to enlarge the capacity of the accommodating space S for excrements. And to form such space Ss, the exterior sheet 8 is bended at the stand-up point Bt as the bending point distant from the absorbent core 9 in the width direction.

However, when the first part 8a positioned at the bending point is not fixed in a case of bending the exterior sheet 8 at the above bending point, the first part 8a shifts so as to approach the absorbent core 9 in the width direction, and the space Ss cannot be appropriately secured. This is because, a part of the exterior sheet 8 distant from the absorbent core 9 has lower rigidity compared to a part in which the absorbent core 9 is positioned (a part on which the absorbent core 9 is placed). That is, because of the difference in rigidity between the two parts, in the case of bending the exterior sheet 8 at the bending point distant from the absorbent core 9, of the exterior sheet 8, a part positioned between the absorbent core 9 and the bending point may shrink or break. Specifically, as it is in the present embodiment, in the case of bending the exterior sheet 8 so that the second part 8b is pulled inward to collapse onto the third part 8c, breaking of the part positioned between the absorbent core 9 and the bending point occurs noticeably.

As a result, as shown in FIG. 14, the first part positioned at the bending point is drawn toward the absorbent core 9 to approach the absorbent core 9, and therefore a sufficient space Ss cannot be secured between the exterior sheet 8 and the absorbent core 9. FIG. 14 is a diagram showing a comparative example for describing the effectiveness of the embodiment.

On the other hand, in the bending process of the present embodiment, when the exterior sheet 8 is bended, the exterior sheet 8 is intimately attached and fixed between the first part 8a and the third part 8c in the width direction to the carrying face 43 of the transportation belt 42 (suppresses misalignment in the width direction), and the approach of the first part 8a to the absorbent core 9 in the width direction is regulated. In this way, even if the first part 8a positioned at the bending point is a part of the exterior sheet 8 with low rigidity, the exterior sheet 8 could be bent at the bending point so as to secure an appropriate space Ss between the exterior sheet 8 and the absorbent core 9. As a result, a function which is to be provided to the exterior sheet 8 (for example, a function of forming the three-dimensional gathered part 13) is suitably exhibited.

### === Regarding Modification Example ===

In the above-mentioned embodiment (hereafter, also referred to as the present example), to regulate the approach of the first part 8a to the absorbent core 9 in the bending process, the suction mechanisms 47 and 48 perform the suction operation so that the exterior sheet 8 is intimately attached to the carrying face 43 of the transportation belt 42 between the first part 8a and the third part 8c in the width direction. Also, to intimately attach the exterior sheet 8 to the carrying face 43 as mentioned above, only the part between the first part 8a and the end part of the third part 8c in the width direction opposes the area in which the air holes 44 are formed to the carrying face 43, while the other parts do not oppose this area. However, there is no limitation to this and other examples can be considered. Hereafter, a first modification example and a second modification example are explained as the other examples that regulate the approach of the first part 8a to the absorbent core 9 in the bending process.

### « First Modification Example »

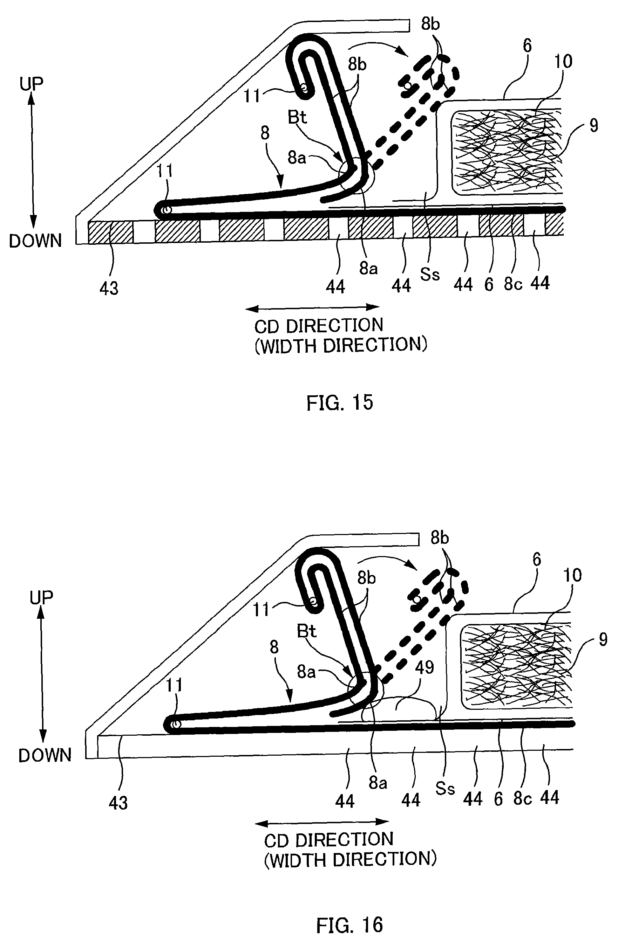

In the first modification example, the suction mechanisms 47 and 48 perform the suction operation to intimately attach the exterior sheet 8 to the carrying face 43 of the transportation belt 42. Up to this point it is same as the present example. However, in the first modification example, as shown in FIG. 15, the air holes 44 are formed on substantially the entire face of the carrying face 43. That is, in the first modification example, the suction pressure acts, of the exterior sheet 8, not only to the part between the first part 8a and the third part 8c but also to the part in which a gathered part at the leg circumference 12 is formed. FIG. 15 is a diagram showing the first modification example.

With such structure, it is possible to bend the exterior sheet 8 at the bending point so that the second part 8b collapses onto the third part 8c while regulating the approach of the first part 8a to the absorbent core 9 (that is, the bending process same as the present example can be performed). Further, in the first modification example, during the bending process, the part where the gathered part at leg circumference 12 is formed is also intimately attached to the carrying face 43, and thus the exterior sheet 8 is bended while preventing such part from fluttering. As above, the first modification example is different from the present example on the point that the suction pressure acts, of the exterior sheet 8, not only to the part between the first part 8a and the third part 8c but also to the part in which the gathered part at leg circumference 12 is formed. However, the other structures are same as the present example and therefore, the first modification example achieves the same effects as the present example.

Note that, in the present example and the first modification example, the air holes 44 are formed on the carrying face 43 of the transportation belt 42 for the suction mechanisms 47 and 48 to perform the suction operation. However, for example, the air holes 44 can be formed by performing a punching process to the transportation belt 42, or using a mesh belt as the transportation belt 42.

### « Second Modification Example »

In the second modification example, a bar-shaped member 49 shown in FIG. 16 is provided instead of the suction mechanisms 47 and 48. FIG. 16 is a diagram showing the second modification example.

The bar-shaped member 49 is an example of a pressing body. In a case where the exterior sheet 8 placed on the carrying face 43 of the transportation belt 42 is bended at the bending point (that is, the stand-up point Bt) the bar-shaped member 49 contacts the exterior sheet 8 (specifically, as shown in FIG. 16, a border of the third part 8c in the width direction and the end part in the width direction) from the skin side. And the bar-shaped member 49 presses the exterior sheet 8 between the first part 8a and the third part 8c in the width direction against the carrying face 43. Thereby, the exterior sheet 8 is pressed against the carrying face 43 and fixed between the first part 8a and the third part 8c in the width direction. That is, it is possible to regulate the approach of the first part 8a to the absorbent core 9 during the bending process.

As above, in the second modification example, shifting of the first part 8a is suppressed by pressing of the bar-shaped member 49 instead of using the suction operation by the suction mechanisms 47 and 48. At this point, the second modification example differs from the present example. However, the other structures are the same as the present example and therefore the second modification example achieves the same effects as the present example. That is, by regulating the approach of the first part 8a to the absorbent core 9 using the bar-shaped member 49, the exterior sheet 8 can be bended so as to appropriately secure the space Ss between the exterior sheet 8 and the absorbent core 9.

Also, a case can be considered in which the exterior sheet 8 is bended at the bending point while a bar-shaped member that is different from the above bar-shaped member 49 (hereafter, other bar-shaped member) is disposed on both sides of the absorbent core 9. The other bar-shaped member does not have a function of pressing the exterior sheet 8 to the carrying face 43, but occupies predetermined spaces on both sides of the absorbent core 9. Therefore, by using the other bar-shaped member instead of the bar-shaped member 49, a space with a width of the other bar-shaped member can be secured on both sides of the absorbent core 9 at the bending process. Thereby, the approach of the first part 8a to the absorbent core 9 during the bending process is regulated, and the space Ss is appropriately secured between the exterior sheet 8 and the absorbent core 9.

### == Other Embodiments ==

In the above-mentioned embodiments, the apparatus for manufacturing a diaper 30 and the method for manufacturing the diaper 1 according to the present invention were mainly explained. However, the above-mentioned embodiments are provided for the purpose of facilitating the understanding of the present invention and do not give any limitation to the present invention. It goes without saying that any modifications and improvements to the present invention can be made without departing from the spirit of the invention and the present invention includes its equivalents. Further, the above-mentioned shapes etc. are merely examples to exert effects of the present invention and should not be understood as any limitation to the present invention.

Also, in the above-mentioned embodiments, the gathered part of leg circumference 12 is formed outside the stand-up point Bt as the bending point. However, there is no limitation to this and the gathered part of leg circumference 12 need not be included outside the bending point. Also, in the above-mentioned embodiments, the back face sheet 7 is interposed between the absorbent core 9 and the exterior sheet 8, and the end part of the back face sheet 7 in the width direction was to reach the stand-up point Bt in the width direction of the exterior sheet 8. However, there is no limitation to this and the back face sheet 7 does not have to be interposed between the absorbent core 9 and the exterior sheet 8, or though the back face sheet 7 is interposed, the end part in the width direction thereof need not reach the stand-up point Bt.

Also, in the above-mentioned embodiments, the gathered part of leg circumference 12 and the three-dimensional gathered part 13 are formed in the exterior sheet 8. However, for example, each of the above gathered parts 12 and 13 can be formed in other sheet besides the exterior sheet 8 (for example, a sheet joined to the skin side of the exterior sheet 8). And the present invention also can be applied to a case where the absorbent main body 2 is placed on the other sheet joined to the exterior sheet 8, and the other sheet is bended at the predetermined bending point to form each of the gathered parts 12 and 13.

Also, in the above-mentioned embodiments, the apparatus and method for manufacturing the absorbent product is explained by using the diaper 1 as an example. However, there is no limitation to this. For example, as other absorbent products there are sanitary napkins, incontinence pads, and wipers or the like, and the present invention can be also applied to the apparatuses and methods for manufacturing these products.

### Reference Signs List

1 diaper (absorbent article), 1a waistline opening, 1b leg circumferential opening, 2 absorbent main body, 3 band (back-side band), 4 band (stomach-side band), 5 absorbent body, 6 surface sheet 6, 7 back face sheet (film), 8 exterior sheet (sheet), 8a first part, 8b second part, 8c third part, 8d overlapping part, 9 absorbent core, 10 tracing paper, 11 stretchable member, 12 gathered parts at the leg circumference, 13 three-dimensional gathered parts, 14 elastic member, 15 continuous body of diapers, 16 a piece of continuous body of diapers, 17 continuous band before separation, 18 continuous body, 19 continuous band, 20 continuous band, 21 arch, 22 complex, 30 apparatus for manufacturing a diaper (apparatus for manufacturing an absorbent article), 31 absorbent main body manufacturing unit, 32 band supplying unit, 33 transfer unit, 34 product cutting unit, 35 splitter, 41 transportation mechanism, 42 transportation belt, 43 carrying face, 44 air hole, 45 bending jig (bending part), 46 bending jig (bending part), 47 suction mechanism, 48 suction mechanism, 49 bar-shaped member (pressing body), Bd fold-back point, Bt stand-up point (bending point), Bk second fold-back point, Bf third fold-back point, S accommodating space, Ss space

## Claims

1. An apparatus for manufacturing an absorbent article, the absorbent article including an absorbent core and a sheet whereon the absorbent core is placed, comprising:
a transportation section transporting in a transport direction the sheet whereon the absorbent core is placed; and
a bending section bending the sheet, in an intersecting direction that intersects the transport direction, while the transportation section is transporting the sheet;
wherein a first part of the sheet, positioned in a bending point where the bending section bends in the intersecting direction the sheet, is distant in the intersecting direction from the absorbent core,
a second part of the sheet positioned on an opposite side of the first part from the absorbent core, in the intersecting direction, collapses onto a third part positioned on the absorbent core side from the first part, in the intersecting direction, by the bending section bending the sheet at the bending point,
the bending section further including a regulation section regulating an approach of the first part to the absorbent core, in the intersecting direction, when the bending section is bending the sheet.

2. An apparatus for manufacturing an absorbent article according to claim 1, wherein
the transportation section transports the sheet on a carrying face whereon the sheet is placed,
the bending section bends at the bending point the sheet placed on the carrying face, and
the regulation section is a suction mechanism that performs suction operation to intimately attach to the carrying face the sheet, between the first part and the third part in the intersecting direction.

3. An apparatus for manufacturing an absorbent article according to claim 2, wherein
the absorbent article has a film positioned between the absorbent core and the sheet in a thickness direction of the absorbent article,
the transportation section transports the sheet in a state where the absorbent core is placed on the sheet with the film therebetween, and
the suction mechanism performs the suction operation with the film provided between the first part and the third part, in the intersecting direction.

4. An apparatus for manufacturing an absorbent article according to claim 1, wherein
the transportation section transports the sheet on a carrying face whereon the sheet is to be placed,
the bending section bends at the bending point the sheet placed on the carrying face, and
the regulation section is a pressing body that contacts the sheet to press the sheet against the carrying face, between the first part and the third part in the intersecting direction.

5. An apparatus for manufacturing an absorbent article according to any of claims 1 to 4, wherein
the first part and another first part are positioned on either end sides of the sheet, the second part and another second part are positioned outside the first parts, and the third part is positioned between the first parts, in the intersecting direction,
the absorbent core is provided on the third part,
two bending sections are provided, where one bending section bends the sheet at a bending point on a one end side so that the second part positioned on the one end side in the intersecting direction, of the two second parts, collapses onto the third part and
another bending section bends the sheet at the bending point on an other end side so that the second part positioned on the other end side collapses onto the third part,
two regulation sections are provided, where one regulation section regulates an approach of the first part positioned at the bending point on the one end side to the absorbent core, in the intersecting direction, when the one bending section bends the sheet at the bending point on the one end side and
another regulation section regulates an approach of the first part positioned at the bending point on the other end side to the absorbent core, in the intersecting direction, when the another bending section bends the sheet at the bending point on the other end side.

6. A method for manufacturing an absorbent article, the absorbent article including an absorbent core and a sheet whereon the absorbent core is placed, comprising:
transporting in a transport direction the sheet whereon the absorbent core is placed; and
bending the sheet, in an intersecting direction that intersects the transport direction, while transporting the sheet;
wherein a first part of the sheet, positioned in a bending point when bending the sheet in the intersecting direction, is distant in the intersecting direction from the absorbent core,
the sheet is bent at the bending point so that a second part positioned on an opposite side of the first part from the absorbent core, in the intersecting direction, collapses onto a third part positioned on the absorbent core side from the first part, in the intersecting direction, when bending the sheet, and
an approach of the first part to the absorbent core, in the intersecting direction, is regulated when bending the sheet.
